## Europäisches Patentamt

## European Patent Office

## Office européen des brevets

(11) Veröffentlichungsnummer: **0 152 091**

**A2**

(12) **EUROPÄISCHE PATENTANMELDUNG**

(21) Anmeldenummer: 85101462.1

(22) Anmeldetag: 11.02.85

(51) Int. Cl.⁴: **C 12 Q 1/40**

(30) Priorität: 11.02.84 DE 3404876

(43) Veröffentlichungstag der Anmeldung:
21.08.85 Patentblatt 85/34

(84) Benannte Vertragsstaaten:
AT BE CH DE FR GB IT LI LU NL SE

(71) Anmelder: GÖDECKE AKTIENGESELLSCHAFT
Salzufer 16
D-1000 Berlin 10(DE)

(72) Erfinder: Witt, Peter, Dr.
Vogelwäldeleweg 6
D-7844 Neuenburg(DE)

(54) Verfahren zur Verbesserung der selektiven Aktivitätshemmung von humaner Pankreas- und Speichelamylase sowie eine hierfür geeignete Inhibitorzubereitung.

(57) Die Erfindung betrifft ein Verfahren zur Verbesserung der selektiven Aktivitätshemmung bei der Analyse von humaner Pankreasamylase zusammen mit humaner Speichelamylase durch Zugabe von Amylase-Inhibitoren zur Probe, das dadurch gekennzeichnet ist, daß man der Inhibitorlösung, bezogen auf 1 U Inhibitor im Bereich von $1 \times 10^{-7}$ bis 10 mMol Guanidin oder Guanidinderivate solange zugibt bis die Aktivität des Inhibitors eine maximale Hemmung aufweist und daß man anschließend die Inhibitormenge so weit erhöht, daß sie bezogen auf den Wert der Pankreasamylase die Ausgangsaktivität wieder erreicht. Weiterhin betrifft die Erfindung eine Inhibitorzubereitung zur Verbesserung der selektiven Aktivitätshemmung von humaner Pankreas- und Speichelamylase, die dadurch gekennzeichnet ist, daß die Amylaseinhibitorlösung bezogen auf 1 U Inhibitor zusätzlich $1 \times 10^{-7}$ bis 10 mMol Guanidin oder dessen Derivate enthält.

EP 0 152 091 A2

Croydon Printing Company Ltd.

- 1 -

Beschreibung

Die Erfindung betrifft ein Verfahren zur Verbesserung der selektiven Aktivitätshemmung von humaner Pankreas- und Speichelamylase.

Unter dem Begriff "$\alpha$-Amylase" werden mehrere Isoenzyme zusammengefaßt, die in Pankreas und Parotis gebildet werden und dem enzymatischen Abbau von Polysacchariden im Gastrointestinaltrakt dienen. Ein geringer Teil der $\alpha$-Amylase gelangt ständig in den Blutkreislauf und von hier über die Nieren in den Urin. Bei Gesunden besteht der Amylasespiegel im Serum bzw. Harn zu etwa 1/3 aus $\alpha$-Amylase pankreatischen Ursprungs und zu etwa 2/3 aus $\alpha$-Amylase salivären Ursprungs.

Bei Erkrankung von Pankreas und/oder Parotis kann die Aktivität der Gesamt-$\alpha$-Amylase im Serum und Urin erheblich ansteigen, wobei sich das Verhältnis Pankreas/Parotisamylase im allgemeinen ändert. $\alpha$-Amylase wird im Serum und/oder Urin vor allem bestimmt, um eine akute Pankreatitis nachzuweisen. Dabei kommt es zu einem starken Anstieg der $\alpha$-Amylase pankreatischen Ursprungs im Serum und Urin. Bei einer Erkrankung der Parotis wird dagegen die $\alpha$-Amylase salivären Ursprungs ansteigen. Somit ist bei der Bestimmung nur der Gesamt-$\alpha$-Amylase eine differenzierte Diagnostik nicht möglich. Es ist somit im Sinne einer verbesserten Differentialdiagnostik von Vorteil, zwischen $\alpha$-Amylase pankreatischen und salivären Ursprungs zu unterscheiden, was durch eine möglichst weitgehende Inhibierung derjenigen Isoamylase erreicht werden kann, deren Aktivität nicht bestimmt werden soll.

In Jap. J. Gastroent. 73/6, S. 668-676 wird beschrieben, daß ein bestimmtes Guanidinderivat als Amylase-Inhibitor wirkt.

Es wurde nun gefunden, daß dies für die meisten Guanidinderivate nicht zutrifft, sondern daß Guanidin und dessen Derivate im Gegenteil sogar die Leistung von im System anwesenden Amylase-Inhibitoren verringern. Beim Versuch den nach Guanidinzugabe erfolgten Leistungsverlust des Inhibitors durch Erhöhung der Inhibitorkonzentration wieder auszugleichen, zeigte sich nicht nur, daß dies ohne weiteres möglich ist, sondern auch daß dabei überraschend eine proportional wesentlich höhere Hemmung der Speichelamylase im Vergleich zur Pankreasamylase eintritt.

Beträgt bei Verwendung eines Inhibitors X die Hemmung der Pankreasamylase beispielsweise 50 % und diejenige der Speichel-Amylase 85 %, so wird die Hemmung bei Zugabe von Guanidin oder von dessen Derivaten für beide Amylasen reduziert. Erhöht man dann die Menge Inhibitor X bis die Hemmung der Pankreasamylase wieder den Ausgangswert von 50 % erreicht hat, so stellt man fest, daß unter diesen Bedingungen die Hemmung der Speichelamylase erheblich angestiegen ist und nunmehr beispielsweise 95 % beträgt.

Damit eröffnet sich ein eleganter Weg, die bekannten Amylase-Tests erheblich selektiver und genauer zu machen.

Bei der bekannten Bestimmung der Pankreasamylasen und Speichel-Amylasen geht man so vor, daß man zunächst beide genannten Amylasen gemeinsam bestimmt. In einem zweiten Testansatz wird ein Inhibitor in einer solchen Menge zugegeben, daß die Pankreasamylase zu 50 % und die Speichelamylase zu 85 % gehemmt wird. Nach Durchführung der Bestimmung wird der Analysenwert für die PankreasAmylase unter Berücksichtigung von Korrekturfaktoren vom Analysenwert der Gesamtamylasen abgezogen, so daß auch der Analysenwert der Speichelamylase erhalten wird.

- 3 -

Erfindungsgemäß gibt man zu dem Inhibitor des zweiten Testansatzes pro 1 U Inhibitor bis zu 10 mMol Guanidin, oder ein Guanidinderivat und stellt dann, wie oben beschrieben die Inhibitorkonzentration so ein, daß die Pankreasamylase zu 50 % gehemmt wird. In einer Testanalyse mit bekannten Amylasekonzentrationen wird man feststellen, daß bei der mit Guanidin bzw. mit dessen Derivaten versetzten Probe die Hemmung der Speichelamylase auf über 95 % bis zu fast vollkommener Hemmung angestiegen ist. Es liegt auf der Hand, daß damit der Analysenwert für die Pankreasamylase erheblich genauer bestimmt werden kann, bzw. daß sich Korrekturfaktoren bei der Ermittlung der Speichelamylase praktisch erübrigen, weil die Fehler, die im Analysensystem liegen so gering geworden sind daß sie in den Bereich der Meßfehlergrenzen fallen.

Gegenstand der Erfindung ist ein Verfahren zur Verbesserung der selektiven Aktivitätshemmung bei der Analyse von humaner Pankreas-Amylase zusammen mit humaner Speichelamylase durch Zugabe von Amylase-Inhibitoren zur Probe, dadurch gekennzeichnet, daß man der Inhibitorlösung, bezogen auf 1 U Inhibitor im Bereich von $1 \times 10^{-7}$ bis 10, bevorzugt $3 \times 10^{-4}$ bis $3 \times 10^{-1}$ mMol Guanidin oder Guanidinderivate solange zugibt bis die Aktivität des Inhibitors eine maximale Hemmung aufweist und daß man anschließend die Inhibitorkonzentration so weit erhöht, daß sie bezogen auf den Wert der Pankreasamylase die Ausgangsaktivität wieder erreicht.

Ein weiterer Gegenstand der Erfindung ist eine Inhibitorzubereitung zur Verbesserung der selektiven Aktivitätshemmung von humaner Pankreas- und Speichelamylase, dadurch gekennzeichnet, daß die Amylaseinhibitorlösung bezogen auf 1 U Inhibitor zusätzlich $1 \times 10^{-1}$ bis 10 mMol Guanidin oder dessen Derivate enthält.

Als Guanidinderivate kommen solche Verbindungen in Frage, welche in ihrer Struktur mindestens ein Guanidinmolekül enthalten und ein Molgewicht unter 50 000 aufweisen. Bestehen die Guanidinderivate in ihrer molekularen Zusammensetzung insgesamt zu mehr als 50 % aus Guanidin, Biguanidin oder Arginin, so sind diese auch oberhalb einer Molgewichtsgrenze von 50 000 erfindungsgemäß aktiv. Es sind auch Komplexe Guanidinderivate wirksam, wie z.B. Protamine vom Typ des Salmins, das 78 % Arginin aufweist.

Umfangreiche Versuche haben gezeigt, daß die Wirksamkeit der Guanidinderivate nur davon abhängt, daß innerhalb der vorgegebenen Grenzen im Molekül die Struktur des Guanidins mindestens einmal vorhanden ist.

Besonders wirksam sind Guanidin oder dessen Derivate mit einem Molekulargewicht von unter 5 000. Besonders bevorzugt sind Guanidinderivate mit einem Molekulargewicht von unter 600.

Als Verbindungen können beispielsweise Guanidin oder dessen Derivate auch in Form von Salzen mit organischen oder anorganischen Säuren eingesetzt werden, wenn sie aufgrund ihrer Basizität solche Salze bilden können. Als Säuren kommen hierbei z.B. folgende in Frage:

Halogenwasserstoffsäuren, wie Chlor- oder Bromwasserstoffsäure, Schwefelsäuren, Phosphorsäuren, Salpetersäure, aliphatischen, alicyclischen, aromatischen oder heterocyclischen Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Brenztraubensäure; Phenylessig-, Benzoe-, p-Aminobezoe-, Anthranil-, p-Hydroxybenzoe-, Salicyl- oder p-Aminosalicylsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Tryptophan oder Lysin.

Werden andererseits Aminosäuren vom Typ des Arginins eingesetzt, können auch dessen Alkali- oder Erdalkalisalze, wie z.B. Ammonium, Natrium, Kalium, Magnesium oder Calciumsalze verwendet werden, oder Salze mit entsprechend starken organischen Basen.

Als Guanidinderivate kommen beispielsweise folgende in Frage:

Biguanid, Argininmethylester, Argininethylester, Kreatin, N-Methylarginin, N-Ethylarginin, N,N'-Diethylarginin, Agmatin, Protamine, wie z.B. Salmin, Guanidinoessigsäure, Guanidinoameisensäure, Guanidinobernsteinsäure, Guanidinobiotin, 2-Guanidinobenzimidazol, 2-Guanidinonitrobenzimidazol, Kreatinin, Guanin, 9-Methylguanin, 9-Ethylguanin, 2-Acetylamino-hypoxanthin oder 2-Methylaminohypoxanthin.

Die Menge des zugegebenen Guanidins oder Guanidinderivates ist relativ unkritisch, da auch ein sehr hoher Überschuß die Reaktion nicht stört. In der Praxis stellt sich oberhalb eines Verhältnisses von $1 \times 10^{-7}$ mMol/U Inhibitor Guanidinverbindung bei einer bestimmten Konzentration die einerseits vom Inhibitor andererseits von den übrigen Testbedingungen abhängt, relativ rasch die erstrebte Inhibitorhemmung ein. Eine über dieses Verhältnis hinausgehende Zugabe von Guanidin hat auf die Inhibitorwirkung dann keinen Einfluß mehr, stört aber auch den Ablauf des Tests nicht. Da die Versuchsbedingungen stark variieren können, ist es empfehlenswert, die optimale Menge innerhalb des Bereiches $1 \times 10^{-7}$ bis 10 m/Mol pro Unit für jedenTest zu ermitteln.

Als $\alpha$-Amylase-Inhibitoren kommen sämtliche Stoffe in Betracht, welche die Aktivität der $\alpha$-Amylasen herabsetzen, insbesondere aus Weizenkeimlingen gewonnene Inhibitoren gemäß Biochem. Biophys. Acta 422, S. 159-169 (1976).

In der Bundesrepublik Deutschland werden fertige Inhibitoren durch die Firma Sigma, München, vertrieben. (z.B. unter Bestell Nr. A 1520)

Eine übliche Inhibitorlösung enthält erfindungsgemäß beispielsweise 50-500 U/ml und entsprechend etwa $1,5 \times 10^{-2}$ bis $1,5 \times 10^3$ mMol/ml Guanidin oder dessen Derivate. Die angegebenen Grenzen sind jedoch nicht bindend. Aus dieser Inhibitorlösung wird pro Test ein aliquoter Teil etwa 100 µl pro 100 µl Amylaselösung für die Bestimmung verwendet. Eine Inhibitor-Unit U ist die Menge Inhibitor, die nach Inkubation bei 25 $^{O}$C die Aktivität von zwei internationalen Einheiten (U) menschlicher Speichelamylasen hemmt.

Die Internationale Einheit (U) der $\alpha$-Amylase ist wie folgt definiert:

1 Internationale Einheit $\alpha$-Amylase setzt 1 mg Maltose innerhalb von 3 Minuten bei 37 $^{O}$C, pH 6,9 vom Substrat Stärke frei.

Die $\alpha$-Amylase-Differenzierung wird im Einzelnen durchgeführt indem die Probe und der Inhibitor 2 - 10 min. aufeinander einwirken, vorzugsweise 3 - 6 min. Danach wird das Substrat zur $\alpha$-Amylase-Bestimmung hinzugegeben. Nach der für die $\alpha$-Amylase-Bestimmung testabhängigen Inkubationszeit wird die Aktivität der $\alpha$-Amylase photometrisch bestimmt.

Beschrieben wird die Durchführung des Tests zur Bestimmung der Pankreas-Isoamylase, wobei die $\alpha$-Amylase in dieser Testbeschreibung nach der Maltotetraose-Methode bestimmt wird. Die Maltotetraose-Methode ist in Clin. Chem. 22, S. 1219 (1976) beschrieben. Bei dieser Methode wird Maltotetraose als Substrat für die $\alpha$-Amylase eingesetzt und nach enzymatischem Maltotetraose-Abbau das entstehende NADH kinetisch bestimmt. Die Konzentration des Inhibitors wird so eingestellt, daß 50 % der Pankreas-Isoamylase und 95 bis 96 % der Speichelamylase gehemmt werden. Die Konzentration der Inhibitorlösung beträgt 95 U/ml Inhibitor und 0,07 mMol/ml Arginin.

Versuchsbeschreibung

Wellenlänge: 340 nm, Hg 334 nm, Hg 365/366 nm Halbmikro- oder Mikroküvetten: 1 cm Schichtdicke

Inkubationstemperatur: 25 $^{\circ}$C

Messung gegen Luft (Extinktionszunahme) Temperatur der Reagenzien: 25 $^{\circ}$C

| In Küvetten pipettieren | |
| --- | --- |
| Probe (Serum oder Urin) <br> Inhibitorlösung [94 U/ml Inhibitor aus Triticum aestivum (Sigma) + 0.07 mMol/ml Arginin] | 100 µl <br> 100 µl |
| Mischen, 5 min. bei 25$^{\circ}$C inkubieren | |
| Amylase-Reagens (Beckmann) | 1,0 ml |
| Mischen nach 10 min. Extinktion messen und gleich-zeitig Stoppuhr starten. Nach genau 1, 2 oder 3 min., Ablesung wiederholen. | |

Aus den Extinktionsdifferenzen pro Minute ($\Delta$ E/min) wird der Mittelwert gebildet. Die $\alpha$-Amylase-Aktivitäten werden mit Hilfe der angegebenen Faktoren berechnet. Da 50 % der Pankreas-$\alpha$-Amylase inhibiert wird muß mit 2 multipliziert werden, um die Pankreas-$\alpha$-Amylase-Aktivitäten zu erhalten. Da die Speichelamylase zu 95 - 96 % inhibiert wird, können die verbleibenden Restaktivitäten ggf. vernachlässigt werden. Eine internationale Einheit (U) ist diejenige Enzymaktivität, die pro Minute unter definierten Bedingungen die Umwandlung von einem µmol Substrat (Maltotetraose) katalysiert.

Berechnung:   $\triangle E/min \times F \times 2 = U/l$

| Wellenlänge | Faktor |
|---|---|
| Hg 334 nm | 971 |
| 340 nm | 953 |
| Hg 365/366 nm | 1765 |

Zur Überprüfung eingesetzt wurden der in Biochem. Biophys. Acta 391, S. 170 (1975) und 422, S. 159 (1976) beschriebene Inhibitor sowie Acarbose C.A. 94, (1981) 187620 h. Bei dem erstgenannten Inhibitor wurde bei 50 %iger Inhibierung der Pankreas-$\alpha$-Amylase die Speichel-$\alpha$-Amylase zu 85 % gehemmt. Werden nun erfahrungsgemäß zu der Inhibitor-Lösung $1 \times 10^{-7}$ bis 10 mMol Guanidin bzw. ein Guanidinderivat gleicher Konzentration, wie z.B. Arginin, Protamin oder Guanidinobenzimidazol hinzugegeben, so beträgt bei 50 %iger Pankreas-$\alpha$-Amylase-Inhibierung die Inhibierung der Speichelamylase bei Guanidin 95 %, beim Arginin 95 %, bei Protaminchlorid 93 % und beim Guanidinobenzimidazol 89 %. Eine gleich gute Wirkung kann durch ein Mischung verschiedener Guanidinderivate erreicht werden.

Bei Verwendung von Acarbose konnte duch Zusatz von Guanidin bzw. Guanidinderivaten die Hemmung der Speichelamylase bei gleichbleibend hoher Hemmung der Pankreas-Amylase erhöht werden, damit von einer gleich hohen Hemmung der Pankreas-Amylase ausgegangen werden konnte. Die Wirkung der Guanidinderivate ist also unabhängig von der Art des verwendeten $\alpha$-Amylase-Inhibitors. Die gesteigerte Hemmung der Speichelamylase konnte sowohl bei Verwendung der Maltotetraose-Methode zur Bestimmung der $\alpha$-Amylase als auch bei Verwendung der verschiedenen anderen $\alpha$-Amylase-Bestimmungsmethoden nachgewiesen werden.

Patentansprüche für BE, CH, DE, FR, GB, IT, LI, LU, NL, SE

1.) Verfahren zur Verbesserung der selektiven Aktivitätshemmung bei der Analyse von humaner Pankreasamylase zusammen mit humaner Speichelamylase durch Zugabe von Amylase-Inhibitoren zur Probe, dadurch gekennzeichnet, daß man der Inhibitorlösung, bezogen auf 1 U Inhibitor im Bereich von $1 \times 10^{-7}$ bis 10 mMol Guanidin oder Guanidinderivate solange zugibt bis die Aktivität des Inhibitors eine maximale Hemmung aufweist und daß man anschließend die Inhibitormenge so weit erhöht, daß sie bezogen auf den Wert der Pankreasamylase die Ausgangsaktivität wieder erreicht.

2.) Verfahren gemäß Anspruch 1, dadurch gekennzeichnet, daß Derivate des Guanidins mit einem Molgewicht unter 5 000 zugefügt werden.

3.) Verfahren gemäß Anspruch 1, dadurch gekennzeichnet, daß Derivate des Guanidins zugefügt werden, die in ihrer molekularen Zusammensetzung zu mehr als 50 % aus Guanidin, Biguanidin oder Arginin bestehen.

4.) Inhibitorzubereitung zur Verbesserung der selektiven Aktivitätshemmung von humaner Pankreas- und Speichelamylase, dadurch gekennzeichnet, daß die Amylaseinhibitorlösung bezogen auf 1 U Inhibitor zusätzlich $1 \times 10^{-7}$ bis 10 mMol Guanidin oder dessen Derivate enthält.

5.) Inhibitorzubereitung gemäß Anspruch 4, dadurch gekennzeichnet, daß er als Derivat des Guanidins ein solches mit einem Molgewicht unter 5 000 enthält.

6.) Inhibitorzubereitung gemäß Anspruch 4, dadurch gekennzeichnet, daß diese als Derivat des Guanidins ein solches enthält, welches in seiner molekularen Zusammensetzung zu mehr als 50 % aus Guanidin, Biguanidin oder Arginin besteht.

Patentansprüche für AT

1.) Verfahren zur Verbesserung der selektiven Aktivitätshemmung bei der Analyse von humaner Pankreasamylase zusammen mit humaner Speichelamylase durch Zugabe von Amylase-Inhibitoren zur Probe, dadurch gekennzeichnet, daß man der Inhibitor-lösung, bezogen auf 1 U Inhibitor im Bereich von $1 \times 10^{-7}$ bis 10 mMol Guanidin oder Guanidinderivate solange zugibt bis die Aktivität des Inhibitors eine maximale Hemmung aufweist und daß man anschließend die Inhibitormenge so weit erhöht, daß sie bezogen auf den Wert der Pankreasamylase die Ausgangsaktivität wieder erreicht.

2.) Verfahren gemäß Anspruch 1, dadurch gekennzeichnet, daß Derivate des Guanidins mit einem Molgewicht unter 5 000 zugefügt werden.

3.) Verfahren gemäß Anspruch 1, dadurch gekennzeichnet, daß Derivate des Guanidins zugefügt werden, die in ihrer molekularen Zusammensetzung zu mehr als 50 % aus Guanidin, Biguanidin oder Arginin bestehen.